# EUROPEAN PATENT APPLICATION

(11) **EP 1 136 551 A1**
(43) Date of publication of application: **26.09.2001**
(21) Application number: 00106438.5
(22) Date of filing: 24.03.2000
(51) Int. Cl.: C12N 15/10, C12Q 1/68

(54) **Direct isolation of transposon insertions tagging transcribed portions of genes**

(71) Applicant: Max-Planck-Gesellschaft zur Förderung der Wissenschaften e.V. Berlin, 80539 München (DE)
(72) Inventor: Yephremov, Alexander Dr. c/o Max-Planck-Institut, 50829 Köln (DE); Saedler, Heinz Prof. Dr. c/o Max-Planck-Institut, 50829 Köln (DE)
(74) Representative: Wibbelmann, Jobst, Dr., Dipl.-Chem.

(57) **Abstract**

The invention concerns a method for the identification of transposon insertions within a transcribed portion of a gene of interest in an organism or tissue of interest, said method comprising the following steps:
- isolating RNA from the organism or tissue of interest;
- hybridizing the isolated RNA to one or more polymer molecule(s) containing nucleobases capable of hybridization with at least a part of the transposon sequence in a chimeric RNA gene transcript;
- performing a reverse transcription reaction primed by said polymer molecules;
- optionally amplifying the cDNA resulting from the reverse transcription reaction;
- analysing the obtained cDNA fragments by one or more of the following methods:
   a) analysing the obtained cDNA fragments for segregation of fragments;
   b) using the fragments as probes or targets in nucleic acid hybridization assays;
   c) sequencing of the obtained cDNA fragments;
various applications of said method as well as kits.

## Description

The present invention concerns a method for displaying and isolating transposon flanking sequences within an organism of interest starting from RNA thereof.

The identification of the insertion of a transposon in a gene of interest opens up a way for cloning the latter by using the known DNA sequence of the transposon as a molecular tag. A particular cloning strategy relies on the finding of a coincidence of the tag with a mutant phenotype in segregating progeny. Accordingly, the insertion mutagenesis using transposons has become a powerful tool for the isolation of relevant genes within a particular biochemical or developmental pathway.

### BRIEF DESCRIPTION OF THE RELATED ART

Up to now, two types of techniques have been used in practice to display the segregation of transposon insertions. Though these approaches are both based on restriction fragment length polymorphisms (RFLPs) which appear in genomic DNA due to transposon insertions and on segregation analysis of DNA fragments in a gel they use different techniques to reveal tag-specific DNA fragments. The first procedure utilizes a Southern blot hybridization with a transposon derived molecular probe (Walbot, 1992) whereas the second approach exploits PCR to amplify DNA sequences flanking transposons (Earp et al., 1990; Frey et al., 1998; Van et al., 1998; Yephremov and Saedler, 1995).

For the latter strategy, similarly to other applications of PCR aimed at the amplification of DNA, first, a PCR template has to be constructed. In principal, this may be achieved either before the PCR by means of circularization of DNA fragments, tailing reactions and ligation of DNA fragments with defined sequences (Ochman et al., 1993) or directly in PCR using a degenerated oligonucleotide as a primer for the flanking sequences (Liu et al., 1995).

The ligation of linkers to digested genomic DNA is used in another PCR based technique, the so-called Transposon Insertion Display (TID), for synthesizing PCR templates (Yephremov and Saedler, 1995). TID protocols were developed for three classes of maize transposable elements -- *Cin4* (Schwarz-Sommer et al., 1987; *Mul* (Bennetzen et al., 1984); and *En/Spm* (McClintock, 1954; Pereira et al., 1986; Peterson, 1953) - and used for the examination of the segregation of transposons and for isolating their flanking sequences in maize, teosinte and *Arabidopsis.* Using the TID protocol several transposon tagged genes have been cloned from *Arabidopsis* populations mutagenized with *En/Spm* transposons.

All the above-referenced methods are, i.a., characterized in that they are directed to the identification of transposon flanking sequences in genomic DNA based on RFLPs in such genomic DNA.

### SUMMARY OF THE INVENTION

The present invention provides a novel approach for the cloning of genes that are tagged by transposons. Compared to all other approaches for the isolation of transposon flanking sequences used so-far, the novel method, called the "Expression Transposon Insertion Display" or ETID technique, is not based on RFLPs in genomic DNA but on the fact that a part of a transposon inserted in a gene may be cotranscribed from a gene promoter to chimeric RNA composed of both transposon transcript portions and gene transcript portions. According to the invention, corresponding chimeric RNA is reverse transcribed by use of one or more primer molecules hybridizing to at least a part of the transposon transcript portion resulting in a reverse transcription - and optional amplification - of at least a part of the chimeric RNA including at least a part of the gene transcript portion. The resulting transcripts are then analysed for segregation of fragments and, optionally, cloned and/or sequenced.

The sequence information obtained for the gene transcript portion may be further utilized, i.a., for the construction of nucleotide probes for the screening of wild type plants, i.e. plants without any mutation of said gene by this particular transposon, for the complete wild-type gene. For this, usually, wild type plants without any mutation by this particular transposon will be utilized.

The ETID method offers important advantages for the isolation of mutant alleles of genes giving expression in specific tissues in plants and animals.

### DETAILED DESCRIPTION OF THE INVENTION

With the present invention, a novel procedure, ETID, is introduced which takes a direct approach for the isolation of transposon insertions tagging transcribed portions of genes. ETID uses RNA as a starting material and exploits primer molecules hybridizing to at least a part of the transposon transcript portion of the RNA for the identification of transcribed transposon copies linked to gene transcripts.

Transposon insertions located in the transcribed portion of a gene generally should abolish gene function and, therefore, are of a great interest. In this kind of mutations, the gene promoter is unaffected by mutation and hence should be transcriptionally active. Expression of the mutant allele should result in chimeric RNA which harbours a portion of the gene transcript at the 5' end that proceeds to transposon sequences which normally are not transcribed in the transposon. The ETID technique is based on the specific reverse transcription - and usually also amplification - of the 5' end portions of these chimeric RNAs followed by an analysis of the amplified fragments, usually by gel analysis.

For the identification of transcript copies comprising, besides transposon portions, parts of gene transcripts, a number of techniques described for the amplification of sequences at the 5' end of transcripts (see, e.g., Schaefer, 1995) may be utilized. The 5' RACE PCR technique (Frohmann et al., 1988) was found to be particularly suitable. At present, the SMART technique (Chenchik et al., 1998) that utilizes an oligo-mediated template switch at the 5' end of the RNA during the reverse transcription reaction is the method of choice. Provided that cDNA synthesis starts with a transposon specific primer and continues into the transcribed portion of the tagged gene and into the SMART oligonucleotide template, resulting fragments acquire defined sequences at their ends and can be readily amplified by PCR.

The ETID technique may employ essentially the same transposon nested primers as are used in the TID technique which are chosen close to the ends of the transposons.

In a preferred embodiment, the method of the invention comprises an additional capturing step of nucleic acids resulting from the use of particular polymer molecules containing nucleobases capable of hybridization with sequences of the transposon and carrying attached labelling molecules which may be specifically bound by particular binding molecules, one example thereof being the combination of biotin as the labelling molecule, and streptavidin or avidin as the binding partner. Alternatively, said polymer molecules containing nucleobases capable of hybridization with sequences of the transposon may themselves be immobilized on a solid carrier phase. The said polymer molecules containing nucleobases should be able to hybridize in a sequence-specific manner to transposon sequences, preferably to those which are not parts of transcripts of the endogenous transposon promoters. As illustrative examples of the said polymer molecules there may be mentioned DNA and RNA molecules such as oligodeoxyribonucleotides, as well as peptide nucleic acids (PNAs). By use of one or more specific polymer molecule(s) containing nucleobases with one or more attached labelling molecule(s), the hybrids resulting from the hybridization of said polymer molecule(s) with total RNA or cDNA may be purified from most of unspecific RNA or cDNA by binding said hybrids to the particular binding molecules immobilized on a suitable solid carrier phase, such as polymeric beads. If specific polymer molecule(s) containing nucleobases are used which are themselves immobilized on a solid carrier phase, the hybridization of said polymer molecule(s) with total RNA or cDNA will lead to an immobilization of the target RNA or cDNA molecules which in form of the resulting immobilized hybrids will be easily separable from any unspecific RNA or cDNA.

The specific polymer molecules containing nucleobases capable of hybridization to at least a part of a RNA transcript of the transposon or a cDNA transcript of the latter will often also be able to prime a reverse transcription reaction. However, there are situations conceivable, where, in the nucleic acid capturing step, the utilization of particular polymeric molecules containing nucleobases capable of a specific hybridization which, however, are not able to prime a reverse transcription reaction, will appear to be more suitable since they may provide for a more specific and/or stronger hybridization and, accordingly, enable a more efficient purification of target RNA or cDNA molecules. Accordingly, corresponding polymeric molecules containing nucleobases, for example, particular PNA molecules, may as well be used in such nucleic acid capturing steps.

In case of the realization of a nucleic acid capturing step with total RNA prior to the reverse transcription reaction, in a next step, reverse transcription may be initiated on selected RNA using the polymer molecules containing nucleobases as primers which have been used for the nucleic acid capturing step. Alternatively, other polymer molecules containing nucleobases specific to transposon sequences may be added as the primers of the reverse transcription reaction; of course, this will always become necessary if using polymer molecules containing nucleobases which are not able to prime a reverse transcription reaction, for the nucleic acid capturing step.

Alternatively, the subsequent PCR reaction may be carried out by use of correspondingly labelled primers enabling the convenient purification of the amplification products after the PCR reaction in a similar way as outlined above.

Accordingly, in a particularly preferred embodiment, the method of the invention may comprise a capturing step of biotinylated nucleic acids resulting from the use of specific polymer molecules containing nucleobases capable of hybridization with sequences of the transposon or the transposon transcript. Accordingly, by means of a selection for hybridization products comprising biotinylated nucleic acids which may be performed at a very early stage in the protocol it becomes possible to get rid of most RNA and DNA fragments in the reaction mixture, respectively, before any amplification starts or immediately after the amplification step. Thereby, it may be assured that essentially all resulting fragments are derived from transposon flanking sequences.

To accomplish this, a first transposon specific primer which is used to start cDNA synthesis in the ETID method may be biotinylated. The resulting transposon specific biotinylated oligonucleotide may be bound to streptavidin immobilized on a solid carrier phase, such as streptavidin coated particles, and hybridized to total RNA to pursue direct capture of hybrids. DNA-RNA hybrids are washed to remove unhybridized RNA and inhibitors (van Doorn et al., 1992). An alternative indirect method comprises first the hybridization of a biotinylated oligonucleotide in solution followed by the addition of streptavidin coated particles. Subsequently, a reverse transcription (RT) reaction may be performed on solid-phase followed by nested PCR and gel separation.

Alternatively, biotinylated primers may be used for the nested PCR reaction, the capturing step then being performed subsequent to the PCR amplification in a similar way as outlined above by use of streptavidin molecules immobilized on a solid carrier phase.

Depending on the abundance of chimeric transcripts, in some cases, conventional 5'-RACE PCR on total RNA may be sufficient; however, in many cases, hybrid selection for transposon sequences will be a method of choice for the ETID technique, since it allows to begin with a large amount of RNA and to perform an additional purification step before the reverse transcription reaction or to perform a convenient purification step after the amplification step.

After the reverse transcription reaction and optional amplification, the fragments will be analysed by known methods that achieve a separation of the said fragments by size such as by gel electrophoresis (agarose gels, polyacrylamide gels, ..) and/or by nucleic acid hybridization. In the latter case, the said fragments may be labelled and used to probe DNA sequences immobilized on a suitable solid carrier material such as filters and microchip matrices; alternatively, they may be immobilized on carrier materials and probed with labelled fragments comprising proper sequences.

When the experiment is directed to the cloning of a mutant transposon insertion allele of a gene which controls a particular phenotype, the correlation of this phenotype with the presence of a particular fragment may be established by means of cosegregation analysis that is regularly done in transposon tagging (Walbot, 1992). For that, at least several individuals segregating the mutant allele have to be subjected to the technique according to the present invention so that the fragment constituting the mutant allele of interest may be identified. In practice, this result has to be confirmed by further cosegregation analysis using DNA gel blot hybridization to explicitely characterize the composition of alleles in the identified genetic locus in a large number of individuals. Therefore, it is necessary to isolate particular candidate fragments obtained by the technique according to the present invention so that hybridization probes may be generated from them. One preferred way to achieve this is to perform a preparative separation of fragments by size, and another one is to clone the bulk of fragments into a standard vector which is able to propagate in bacterial or eucaryotic cells and to screen for clones containing the appropriate insert.

In a particular embodiment of the invention, the segregation analysis of labelled fragments is done under denaturing conditions in a polyacrylamide gel (PAAG). After identification of the fragments of interest, a preparative gel separation is performed, the fragments of interest are located, cut out and usually reamplified for sequencing.

In order to improve the method according to the present invention in particular cases, a use of concentrated guanidine hydrochloride and guanidine thiocyanate solutions as the hybridization buffers may be helpful. Additionally, end labelling and separation of ETID products under denaturing conditions in PAAG may aid in the identification of minor bands compared to agarose gels. Also, to favor a production of longer fragments in ETID, primers containing inverted terminal repeat sequences at their 5' ends may be used in PCR similarly to what was described by Shagin et al., 1999.

In case that the experiment is not directed to the cloning of the particular gene but rather to a high-throughput identification of insertion knock-out alleles of genes, the said fragments may be used as probes or targets in hybridization assays as outlined above rather then for cosegregation analysis. Alternatively, they may be isolated and processed for direct sequencing.

As will be illustrated in detail in the examples, several insertion alleles were identified and isolated by the ETID technique by use of a population of *Arabidopsis* plants bearing the transposable *En/Spm* element of maize (Wisman et al., 1998).

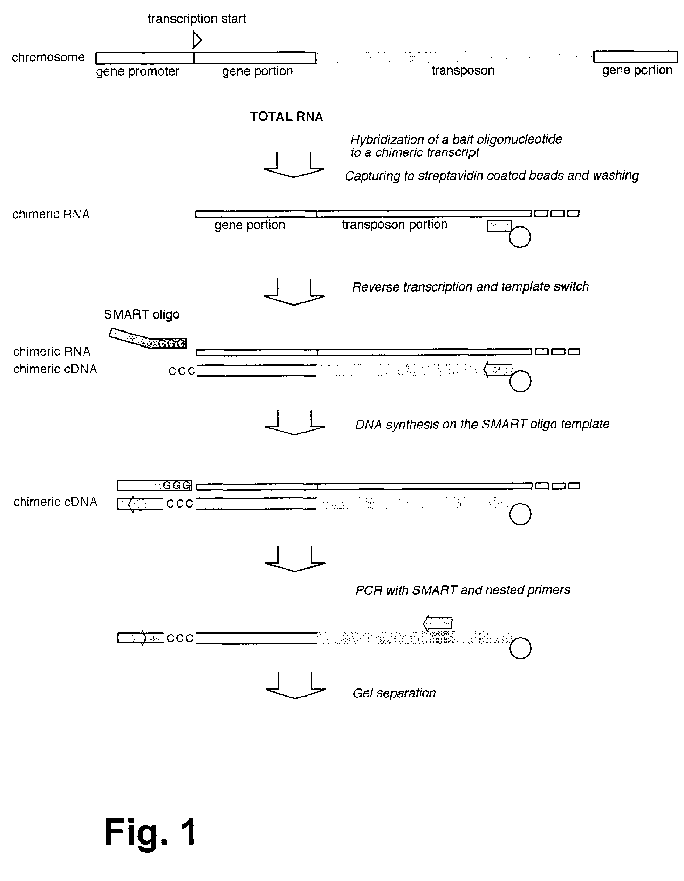

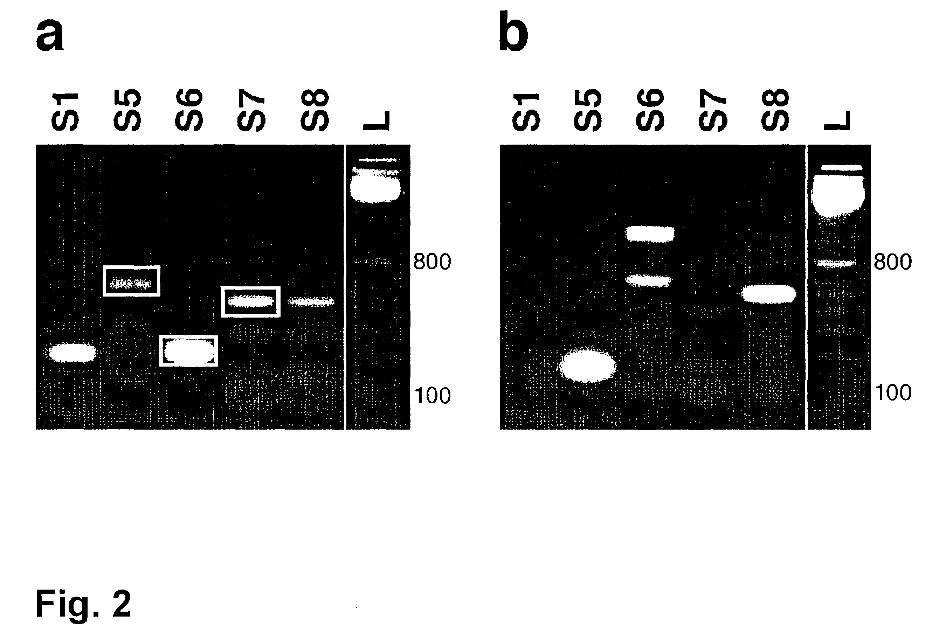

In order to investigate how ETID could be applied to the isolation of genes tagged by transposons, the inventors analysed BC₂S selfing progeny of BC₂ plants that were used in the cloning of *DWF4* (Choe et al., 1998). Vegetative and floral tissues of about 20 wild type looking plants at the beginning of bolting in each BC₂S family were combined for total RNA isolation. After ETID some PCR products were visible as prominent bands on an agarose gel (Figs. 2A, B). The bands were cut out and the sequencing of 3 fragments confirmed that all were flanking transposon sequences. One of them, the C5 fragment of 360 bp, constituted a portion of a novel receptor-kinase gene with the highest sequence similarity to GenBank accession AC005957 which encodes a putative disease resistance protein in *Arabidopsis.* This result was confirmed by the sequencing of a 1,2 kb DNA fragment flanking *En/Spm* at the opposite side of this insertion.

The second fragment, C78, of 278 bp aligned to a chromosome 4 sequence of the F23E12 BAC clone (GenBank accession AL022604) at nucleotide positions 72480-72751. Interestingly, according to the database information, a computer analysis did not predict that this region being a part of a transcript. The ETID results strongly suggest that RNA transcription starts on the opposite strand at the position corresponding to nucleotide 72751 of this clone.

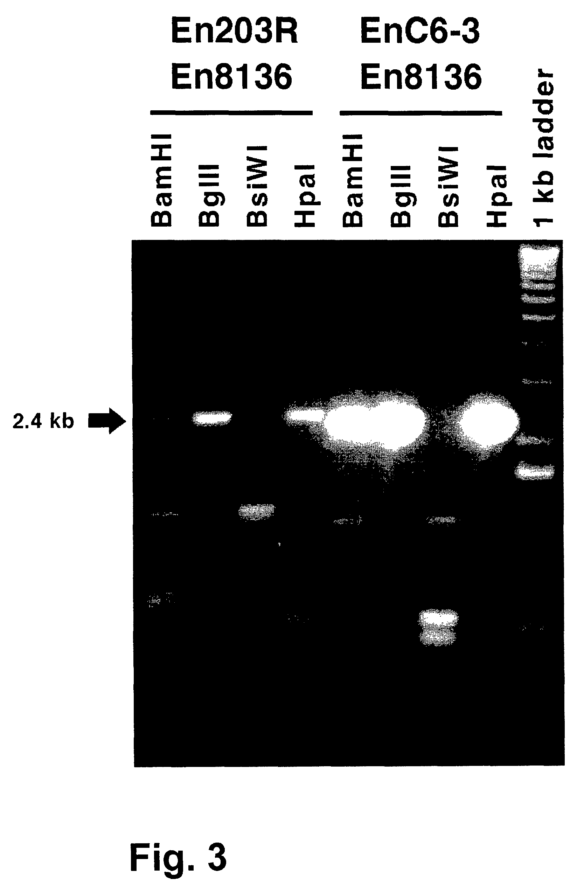

A very short flanking sequence of the third fragment of 31 bp, designated C6, did not allow to perform reliable homology searches in databases. A PCR analysis has demonstrated that the corresponding *En/Spm* insertion is present in the BC₂ parental plant. In order to isolate a larger portion of the C6 fragment BC₂ genomic DNA was subjected to inverse PCR (Earp et al., 1990) with nested primers taking advantage of the known sequence of the C6 fragment (see the examples). The addition of only three C6-specific nucleotides at the 3'-end of the EnC6-3 primer resulted in the preferential amplification of the 2.4 kb fragment as compared to the combination of nested transposon primers (Fig. 3). The fragment was isolated and sequenced, and this allowed to identify the corresponding gene as a novel homolog of late embryogenesis abundant (LEA) proteins, particularly of *Arabidopsis* ECP63 (Yang et al., 1997).

Since the ETID technique is a direct approach for the isolation of transposon insertions that tag transcribed portions of genes, it effectively limits the number of candidate transposon insertions that have to be subjected to a cosegregation analysis with a mutant phenotype.

Provided RNA is isolated from a particular organ or tissue which features are affected in a mutant, the ETID technique enables that the number of transposon insertions which has to be checked may be further reduced. This will significantly reduce the number of candidate transposon insertions to be checked when compared to the TID technique or Southern blot hybridization.

The ETID technique allows to identify knock-out insertion alleles in heterozygous loci. For this, heterozygous wild type organisms may provide tissues which do not develop properly in mutants. Accordingly, even lethal insertion alleles may be exposed by the ETID technique. This represents a particular advantage for developmental mutants.

Accidentally, the ETID technique may result in the identification of knock-out insertion alleles of genes which mutants do not manifest a phenotype distinguishable by the naked eye from that of wild types. Such alleles may also be found in reverse genetic screens; however, compared to this approach, the ETID technique does not require knowing gene sequences.

Although, in the course of the investigations with regard to the present invention, it has been found that the ETID technique is very effective in the wild plant *Arabidopsis thaliana*, it is understood that its advantages over DNA-based techniques will be particularly noticeable in species which organisms have larger organs and which large genomes hinder or complicate their complete sequencing. Accordingly, besides bacteria, species to which the ETID technique may particularly be applied include plants including fungi and animal species. Some of such species, for example plants like maize *(Zea mays*), snapdragon *(Antirrhinum majus*), petunia *(Petunia hybrida*), harbor numerous active transposons while other species may have to be mutagenized by characterized transposable elements heterologous to those latter species.

As illustrative examples of plant-specific transposons, there may be mentioned the transposable elements *En/Spm*, *Mu1*, *Cin4, Ac/Ds*, *Mu2 - Mu9*, *Tag1* and *Tam1 - Tam9.* However, it is evident to the skilled scientists that this list includes only a few examples of plant-specific transposons and that, as has been outlined above, the ETID technique may be applied to, e.g., animal- or fungus-specific transposons as well.

Transposable elements that move via DNA intermediates are often integrated in transcriptionally active regions of the genome and are widely used for mutagenesis in plants, invertebrates such as *Caenorhabditis elegans* and *Drosophila* (Bellen et al., 1989; Spradling et al., 1995), and bacteria. The recent discovery of a corresponding type of transposons in vertebrates including many mammals, and reports on their transposition in transgenic mice open up a possibility to establish general transposon-tagged mutagenesis schemes for many animal model species. For example, the *Sleeping Beauty* (SB) transposable element, a reconstructed version of a *Tc1/mariner*-like transposon isolated from fish, is active in human, frog and mice cells (Plasterk et al., 1999). Therefore, unlike the P-element which does not transpose outside the genus *Drosophila,* the SB transposon may allow to mutagenize various heterologous host species. Taking into account that mutagenesis in mice has become the superior approach for establishing genotype-phenotype relationships, and that transposon mutagenesis in many organisms has proven to have advantages compared to a chemical mutagenesis for the ease of identification of resulting mutations, the RNA-based ETID technique may be useful, for example, in mutant mice, other model vertebrates such as zebrafish (*Danio rerio), Xenopus,* and invertebrates as soon as suitable transposition systems will become available.

Many transposable elements that move via RNA intermediates, so-called retrotransposons, were shown to be located outside transcriptionally active regions of genes and even in the heterochromatin. However, in principle, at least some of them, such as the *Tnt1* retrotransposon from tobacco *(Nicotiana tabacum)* and the *Tos17* retrotransposon from rice, may be successfully used for transposon mutagenesis including heterologous hosts (Hirochika et al., 1997; Lucas et al., 1995; Sato et al., 1999). The ETID technique according to the present invention will allow to facilitate the analysis of insertion mutations caused by this class of transposable elements as well. Accordingly, in the context of the present invention, the meaning of the term "transposon" will always include retrotransposons as well.

In addition to transposons in the narrow sense as it is understood due to the scientific definition, and retrotransposons, for example, insertions made in plant chromosomal DNA by integration of *Agrobacterium* T-DNA may also be used for mutagenesis in those plant species that are amenable to genetic transformation. So far, this approach has been intensively applied to *Arabidopsis* and multiple mutants have been generated by these means. Accordingly, in the context of the present invention, the term "transposon" is understood in its broadest sense and covers all kinds of transposable elements and genetic insertion elements including also the T-DNA from *Agrobacterium tumefaciens*. Accordingly, in a like manner, the ETID technique may be used to analyse T-DNA insertions that are cotranscribed from endogenous chromosomal promoters.

For defining the term "plants" in general when used for defining a starting material for providing the RNA to be investigated by means of the ETID technique according to the present invention, this term shall include natural plants, hybrid plants as well as transgenic plants. In the context of the present invention, the term "plants" will always be understood to include fungi. Furthermore, this terms comprises mature as well as immature whole plants and developing plantlets.

The term "natural plants" shall include naturally occurring wild plants (wild-type plants) and cultivated plants obtained by a conventional plant breeding process.

The term "hybrid plants" shall mean plants which are obtained by hybridization, i.e. a genetic recombination process between genomes of plants which are derived from different populations and which cannot be stably propagated without losing some traits.

The term "transgenic plant" shall mean plants which already contain at least one foreign DNA sequence, a so-called "transgene", that is normally not observed in said plants or, alternatively, which is normally observed in a different genetic environment within the plant. The genetically modified plants according to the present invention may also comprise additional genetic modifications, e.g. transgenes conferring resistance against pests or plant protection agents or leading to improved yield.

Plants of interest according to the present invention are all monocotyledonous and dicotyledonous plants, and particularly plants of agricultural, horticultural, floricultural, silvicultural or fruit growing interest, e.g. crops, ornamentals, and trees including ornamental trees.

RNA to be investigated by means of the ETID technique according to the present invention may also be prepared from a particular part of a plant (including fungi). As used in the present specification, the term "part of plants" shall include highly differentiated plant parts such as plant organs, e.g. roots, leaves, etc.; propagating material, e.g. seeds, seedlings, etc., as well as poorly differentiated plant parts such as particular plant tissues, callus, plant cells or plant protoplasts.

Furthermore, the ETID technique will also be applicable to animal species, including primates and humans, where genetic means of segregation analysis are limited, as well as any biological material of animal origin. As an example, P-element insertions in the genome of *Drosophila melanogaster* may be mentioned.

Analogously to the definition of the term "plants", the term "animal species" shall cover natural animal species including animal species resulting from a conventional breeding process, hybrid animal species as well as transgenic animal species. The term "animal species" shall cover any kind of animal species, invertebrates as well as vertebrates including mammals and, particularly, primates and humans. Furthermore, this term shall include animal species in any stage of their development, including the fetal stage.

As mentioned above, RNA to be investigated by means of the ETID technique according to the present invention may also be prepared from any biological material of animal origin. As used in the present specification, the term "biological material of animal origin" shall include highly differentiated as well as poorly differentiated biological materials such as mature tissues; blood; organs, such as heart, kidney, spleen or liver; fetal tissues; the various types of cells or protoplasts.

As used in the present specification, the term "nucleobase" shall include heterocyclic nitrogenous bases, particularly purines or pyrimidines (i.e., adenine, cytosine, thymine, guanine and uracil) which are natural constituents of nucleic acids where they occur in N-glycosidic linkage with a sugar, particularly a pentose. This term shall also include heterocyclic nitrogenous bases, particularly purines or pyrimidines which constitute or form part of not naturally occurring synthetic polymeric molecules such as peptide nucleic acids (PNAs) which comprise a polyamide backbone with nucleobase sidechains. A sequence specific hybridization of nucleic acids and "nucleobase" molecules forming part of corresponding not naturally occurring synthetic polymeric molecules such as PNAs occurs through Watson-Crick-H-bonding between the complementary nucleobases of the nucleic acids and the synthetic polymeric molecules, respectively.

In the present context, the term "primer" shall include a short polymer molecule containing nucleobases, one example being an oligodeoxyribonucleotide complementary to a given nucleic acid sequence and acting as the nucleating point from which the polymerase-mediated enzymatic replication proceeds.

Under a further aspect, the invention pertains to a kit assembling components required for performing the ETID technique. In particular embodiments, the kit according to the present invention may comprise
- one or more primers specifically hybridizing to portions of a particular type of inserted transposon (including retrotransposons and other transposable elements), such as *En/Spm* and/or *Mu* primers; and/or
- one or more transposable element(s) for the mutation of a target organism to be investigated with respect to the insertion of said transposable element(s); and/or
- reagents required for performing the specific reverse transcription and, optionally, also amplification of the 5' end portions of the chimeric RNAs, such as reagents required for performing 5' RACE-PCR; and/or
- reagents required for the analysis for segregation of fragments and/or for the hybridization, cloning and/or sequencing of the obtained fragments;
- one or more auxiliary components, such as buffers, to be used in the ETID method of the present invention.

For the particular embodiment of the claimed ETID technique including a nucleic acid capturing step, the kit may additionally comprise reagents required for performing said nucleic acid capturing step, such as (a) one or more polymer molecules containing nucleobases hybridizing to at least a part of a RNA transcript of the transposon or a cDNA transcript of the latter wherein said polymer molecules additionally carry at least one labelling molecule (such as biotin) which may be specifically bound by a capturing molecule (such as avidin or streptavidin) immobilized on a solid carrier phase and/or (b) one or more polymer molecules containing nucleobases hybridizing to at least a part of a RNA transcript of the transposon or a cDNA transcript of the latter wherein said polymer molecules are immobilized on a solid carrier phase. In case (a), the kit according to the present invention may further contain a preparation of said capturing molecule(s) immobilized on a solid carrier phase. As already mentioned above, the polymer molecules containing nucleobases used for the nucleic acid capturing step will not necessarily also be capable of priming a reverse transcription reaction.

In the kits of the invention, any component may be provided combined with one or more of the other components if applicable, or in separate form.

In the following, the invention will be illustrated by reference to several figures and examples. It is to be understood that the invention is by no means limited to the particular embodiments illustrated in said figures and examples.

### EXAMPLES

### Materials

### Plant material

For the experiments, *Arabidopsis thaliana* plants of ecotype Columbia from a population mutagenized with *En/Spm* (Wisman et al., 1998) were grown from seeds in greenhouses and growing chambers. Families of plants segregating recessive dwarf mutant 2AAL45 were taken for the ETID analysis.

### RNA and DNA isolation

RNA was isolated from aerial parts of *Arabidopsis thaliana* plants with the Total RNA Isolation Reagent (BIOMOL, Hamburg, Germany) and dissolved in formamide (0.5 ml for 1 g of fresh tissue) (Chomczynski, 1992). *Arabidopsis thaliana* genomic DNA was isolated with the Nucleon PhytoPure Plant DNA Extraction Kit (Amersham Pharmacia Biotech, Braunschweig, Germany) according to manufacturer's protocol with modifications to improve the purity. Particularly, the DNA containing supernatant was extracted with a phenol:chloroform mixture (1:1) and chloroform after the first centrifugation before removing the polysaccharides with the Nucleon matrix. A nucleic acid pellet obtained with NaOAc and isopropanol was dissolved in TE (0.2 ml for 1 g of starting tissue) and the solution was treated with RNAse before the final precipitation in the protocol. Fast DNA isolation for PCR analysis was performed as described elsewhere (Edwards et al., 1991).

### Oligonucleotides and linkers

Most of the oligonucleotides and linkers were synthesized on a Model 392 DNA/RNA Synthesizer (Applied Biosystems, Foster City, CA, U.S.A.), eluted with ammonia and purified by gel filtration via Sephadex G-25 NAP10 columns (Pharmacia, Freiburg, Germany). Biotinylated oligonucleotides were obtained from Life Technologies (Neu-Isenburg, Germany) and some unlabelled oligonucleotides were obtained from MWG-Biotech (Ebersberg, Germany).

### En/Spm ETID

Sequences of *En/Spm* transposon primers were:

En205 R          (AGAAGCACGACGGCTGTAGAATAGGA)

BioEn205R   bio(AGAAGCACGACGGCTGTAGAATAGGA)

En203R (AGCACGACGGCTGTAGAATAGGATTG)

En91R (TGCAGCAAAACCCACACTTTTACTTC)

En48R (TTTACTTCGATTAAGAGTGTCGGCC) at the 5′ end;

En8130 (GAGCGTCGGTCCCCACACTTCTATAC)

BioEn8130    bio(GAGCGTCGGTCCCCACACTTCTATAC)

En8133 (CGTCGGTCCCCACACTTCTATACGAAT)

En8136 (CGGTCCCCACACTTCTATACGAATAAGA)

En8179          (TGACGGCTAAGAGTGTCGGTCAAC)  at the 3' end.

The SMART II oligonucleotide (AAGCAGTGGTAACAACGCAGAGTACGCGGG) and a PCR primer (AAGCAGTGGTAACAACGCAGAGT) were components of the SMART PCR cDNA Synthesis Kit (Clontech, Palo Alto, CA, U.S.A.).

### PCR components

Taq polymerase and 10 x PCR buffer were obtained from Roche Molecular Biochemicals (Mannheim, Germany) and dNTPs from Pharmacia. PCR reactions were performed in UNOblock (Biometra, Goettingen, Germany) or PCT-200 (MJ Research, Watertown, MA, U.S.A.) thermocyclers.

### Methods: ETID

### Binding bait oligonucleotides Bio-En205 or Bio-En8130 and hybridization in ETID

200 pmol (2 µl) of a biotinylated oligonucleotide were bound to 1 mg of Dynabeads M-280 Streptavidin paramagnetic particles (Dynal, Oslo, Norway) for 15 min at RT in 22 µl of BAITBIND buffer (20 mM Tris-HCl, pH 7.5, 1 M NaCl), prepared for RNA work according to manufacturer's instructions (Dynal) and resuspended in 65 µl of the BAITBIND buffer. Resulting beads are called hereafter Dynabeads-BBO (Biotinylated Bait Oligonucleotides).

80 µg of total RNA were precipitated from formamide with 4 volumes of EtOH (Chomczynski, 1992), solubilized in 80 µl of a buffer containing 75 µl H₂O, 4 µl 1 M Tris-HCl, pH 7.5, and 1 µl of RNAsin (40 U/µl), and 2 µl of 20% SDS were added after the solubilization. 60 µl of Dynabeads-BBO in the BAITBIND buffer were combined with 60 µl of 20 x SSPE and transferred to the RNA solution resulting in a hybridization buffer of 20 mM Tris-HCl, pH 7.5, 6 x SSPE, 0.2% SDS, 0.2 U/µl RNAsin. The tubes were heated for 3 min at 65°C and incubated for 3 h at 30°C with occasional shaking.

Washing was performed two times with the WASH buffer (20 mM Tris-HCl, pH 7.5, 0.15 M LiCl, 2 mM EDTA, 0.2 U/µl RNAsin and two times with Reverse Superscript II 1^{st} strand buffer (RT buffer) (Life Technologies, Neu-Isenburg, Germany).

### Reverse transcription reaction and PCR

RT reactions were performed under conditions closely similar to those specified in the SMART PCR cDNA Synthesis Kit (Clon-tech, Palo Alto, CA, U.S.A.). After washing, the Dynabeads bearing DNA-RNA hybrids were collected by use of a magnet and resuspended in 20 µl of a buffer containing 0.5 µl RNAsin from a 40 U/µl stock, 2.0 µl of 10x RT buffer, 2 µl of dNTPs from a 10 mM stock, 2 µl of DTT from a 20 mM stock, 10.5 µl of H₂O, 2 µl of SMART II oligonucleotide from a 10 µM stock and 1 µl of Superscript II RT enzyme from a 200 U/µl stock. Tubes were incubated for 10 min at room temperature, then for 1 hour at 42°C with occasional gentle shaking and, finally, for 30 min at 48°C.

The first PCR was performed in 50 µl using EXPAND High Fidelity PCR System components (Roche Molecular Biochemicals), the SMART II primer and a corresponding *En/Spm* transposon primer at concentrations of 0.2 µM each. One reaction volume contained 32.2 µl of H₂O, 5 µl of EXPAND 10x buffer, 5 µl 10x dNTPs from a 2 mM stock, 1 µl of the SMART II primer from a 10 µM stock, 1 µl of a transposon primer, and 0.8 µl of EXPAND polymerase. To this, 5 µl of carefully resuspended RT reaction on Dynabeads-BBO were added. The first PCR program for the PCT-200 equipment (MJ Research) was: 85°C for 2 min, 20 cycles (94.5°C for 35 s, 60°C for 35 s, 68°C for 3 min), 72°C for 3 min. The second PCR was performed in 50 µl containing 35.75 µl of H₂O, 5 µl of 10x PCR buffer (Roche Molecular Biochemicals), 5 µl of 10x dNTPs, 1 µl of the SMART II primer from a 20 µM stock, 1 µl of a transposon primer from a 20 µM stock, 0.25 µl of Taq polymerase (Roche Molecular Biochemicals), and 2 µl of the 1^{st} PCR reaction on Dynabeads-BBO. The second PCR program was: 85°C for 2 min, 20 cycles (94.5°C for 33 s, 60°C for 1 min, 72°C for 3 min), 72°C for 3 min. Transposon primers for the 5' end and the 3' end were En205R and En8130 in the 1^{st} PCR and En203R and En8133 in the 2^{nd} PCR, respectively.

The third PCR before loading on an agarose gel was done with the En91 or En8136 primers using 2-5 µl of the reaction mixture resulting from the 2^{nd} PCR under conditions identical to the 2^{nd} PCR. For the better separation of small fragments, a 2.5% gel was prepared from Small Low Melt Agarose (Biozym, Hameln, Germany). Up to 25 µl of each reaction was loaded onto the gel and run at 5 V/cm in 1 x TAE buffer (40 mM Tris-acetate and 10 mM EDTA). Fragments were cut out of the gel and purified for sequencing with the QIAquick Gel Extraction Kit (Qiagen, Hilden, Germany).

A precise segregation analysis of fragments was made possible by their radioactive labelling in the 3^{rd} PCR and separating them on a 6% sequencing polyacrylamide gel. For this, labelling of En48R and En8179 transposon primers was performed with [γ³²P]ATP. One labelling reaction contained 1 µl (10 pmol) of primer, 0.8 µl of 10x polynucleotide kinase buffer (New England Biolabs, Frankfurt/Main, Germany), 6 U of T4 polynucleotide kinase (New England Biolabs), and 5 µl of [γ³²P]ATP (Amersham Pharmacia Biotech, Braunschweig, Germany). To perform the labelling, the tube was incubated at 37°C for 35 min, and then at 75°C for 10 min to inactivate the enzyme. Radioactive products were obtained in the 3^{rd} PCR in a 10 µl volume. For 10 reactions, the following components were mixed: 76.7 µl of H₂O, 8.4 µl of 10x PCR buffer (Roche Molecular Biochemicals), 8.4 µl of 10x dNTPs, 0.6 µl of a labelled transposon primer, 0.5 µl of Taq polymerase (Roche Molecular Biochemicals). Products of the 2^{nd} PCR in 1.6 µl were added to 8.4 µl aliquots of this mixture and overlaid with paraffin oil before starting the PCR. The PCR program was: 85°C for 2 min, 15-25 cycles (94°C for 35 s, 60°C for 1 min, 72°C for 1 min 30 s), 72°C for 3 min. After completion of the PCR, 6 µl of Stop Solution (10 mM NaOH, 95% formamide, 0.05% bromophenol blue, 0.05% xylene cyanol) were added and the mixtures were transferred to new tubes. The reactions were heated at 70°C for 2 min for denaturation of the DNA and 2-4 µl aliquots were loaded on a sequencing gel.

To prepare 50 ml of a 6% sequencing gel solution, 24 g of urea were dissolved in a mixture containing 15 ml of H₂O, 7.5 ml of a 40% solution of an acrylamide stock (38% acrylamide and 2% bis-acrylamide), and 10 ml of 5x TBE buffer, pH 8.8 (54 g Tris, 15 g boric acid, 4.65 g EDTA in 1 L of H₂O). The volume was adjusted to 50 ml with H₂O and filtered. The supporting glass plate was pretreated with 0.4% Silane A 174 (Merck, Darmstadt, Germany) in an ethanol:acetic acid (100:3) solution to ensure binding of the gel. The notched glass plate to be removed after gel electrophoresis was coated with dimethyldichlorosilane (Merck) using a 10% solution in hexane. The plates were assembled with 0.25 - 0.4 mm spacers, fixed with clamps and placed horizontally for gel casting. Gel polymerization was initiated by the addition of 0.25 ml of 10% ammonium persulphate and 27 µl of TEMED. The solution was poured between the plates using a syringe equipped with a rubber tube and a cut yellow pipettors tip (200 µl-tip). A comb was inserted and the gel was allowed to polymerize for 40 min prior to removing the comb and pre-electrophoresis.

Gels were run in 1x TBE buffer under high-voltage conditions, with a constant amperage of 22-24 mA and a power of about 40 W. After electrophoresis, gels were fixed with 15% acetic acid for 40 min, dried at 80°C and exposed to X-ray films. Preparative gels having wider slots were covered with plastic foil without fixation and drying whereupon they were exposed at -70°C with a properly marked and oriented X-ray film. Bands of interest were cut as gel slices from the preparative gels and eluted overnight in 50-100 µl of TE. DNA was precipitated using 10 µg of linear acrylamide as carrier (Gaillard and Strauss, 1990) and dissolved in 15 µl of TE. Elution steps were monitored with a counter.

Fragments were reamplified under conditions similar to that of the last ETID PCR using 1/5 - 1/3 of the final elution volume. Before sequencing, they were purified in a 2.5% Small Low Melt agarose (Biozym) gel and by using the QIAquick gel extraction kit (Qiagen).

Alternatively, an agarose gel electrophoresis was used for segregation analysis of DNA fragments instead of a polyacrylamide gel. A special grade agarose was used to improve the separation of short DNA fragments. A 2.5% agarose gel containing 0.2 - 0.4 µg/ml ethidium bromide was prepared from Small Low Melt agarose (Biozym) in 1x TAE buffer (10x TAE buffer contains 48.4 g of Tris, 11.4 g of glacial acetic acid, and 20.0 ml of 0.5 M EDTA (pH 8.0) in 1 L of H₂O). Samples were applied in a loading buffer (6x loading buffer contains 15% Ficoll Type 4000, 120 mM EDTA, and 0.25% xylene cyanol), and an electrophoresis was carried out at 4 V/cm in 1x TAE. Bands were visualized under UV light and cut out. The DNA fragments were purified with the QIAquick gel extraction kit (Qiagen) and sequenced directly if their amount was sufficient. In other cases, an additional preparative amplification of DNA fragments was necessary whereupon gel separation and purification procedures were performed. Optionally, DNA was concentrated before sequencing by using the Microcon 100 or Microcon 30 ultrafiltration devices (Amicon, Beverly, MA, U.S.A.).

Sequences of fragments were obtained manually using the *fmol* DNA Cycle Sequencing System (Promega, Mannheim, Germany) or via the institutional service unit. In manual sequencing, polyacrylamide gels prepared as described above were used.

### Determination of the En/Spm flanking sequences in the locus corresponding to the C6 fragment by using inverse PCR

Aliquots of corresponding genomic DNA (300 ng) were digested in 50 µl with Apal, ClaI, MluI, NaeI, NcoI and XhoI which do not cut in the transposon, combined, extracted with phenol:chloroform (1:1) and precipitated with isopropanol using 20 µg of linear acrylamide as a carrier (Gaillard and Strauss, 1990). The DNA fragments were then separated on a 0.6% agarose gel and a portion of the gel corresponding to fragments from 8 kb to 14-20 kb was cut out. After purification from the gel with the QIAprep Spin Gel Purification Kit (QIAGEN) the fragments were circularized overnight at 12°C with T4 ligase in 500 µl and concentrated with the Microcon 100 ultrafiltration device (Amicon, Beverly, MA). Aliquots thereof were digested separately in 20 µl volumes with BamHI, BglII, BsiWI and HpaI restriction enzymes which cut in the transposon portion. 1 µl of each reaction mixture was taken and subjected to 20 cycles of long PCR (EXPAND High Fidelity PCR System, Roche Molecular Biochemicals) with the En205 and En8130 transposon primers. The amplification profile of the long PCR in 25 µl was as follows: 85°C for 2 min, 8 x (94°C for 30 sec, 56°C for 30 sec, 68°C for 4 min), 12 x (94°C for 30 sec, 56°C for 30 sec, 68°C for 4 min plus time extension for 20 sec at each cycle), 68°C for 10 min. The primers were used at 0.3 µM as recommended by the manufacturer. 2 µl were taken for a second long PCR of 20 cycles at the same conditions except that the En203 and En8136 transposon nested primers or the En203 and EnC6-3 (CTCCTTACCTTTTTTCTTGTAGTGGCT) nested primers were taken. The sequence of the EnC6-primer was derived from that of a junction between the 3' end of the *En/Spm* insertion (underlined) and genomic DNA at the C6 locus. The combination of transposon nested primers allowed to amplify multiple *En/Spm* flanking sequences while the En203 and EnC6-3 primers selectively amplified the C6 insertion fragment. The PCR products were separated on a 0.8% agarose gel. The same fragment of 2.4 kb was apparently amplified in BamHI, BglII and HpaI samples with the En203 and EnC6-3 primers, suggesting that it contains the C6 insertion (Fig. 6).

### FIGURE LEGENDS

Figure 1
   Identification of transposon insertions tagging transcribed portions of genes by ETID
   Transcripts containing transposon sequences are selected by hybridization with a biotinylated oligonucleotide and used for 5' RACE PCR. 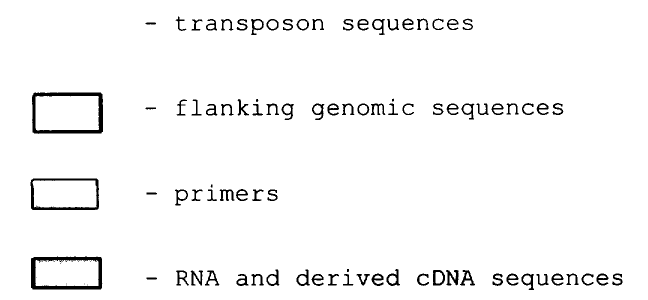
- transposon sequences

 - flanking genomic sequences

 - primers

 - RNA and derived cDNA sequences
 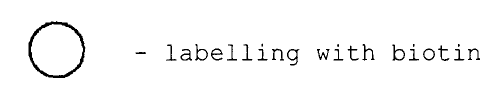
Figure 2
   Amplification of expressed *En/Spm* transposon flanking sequences by ETID in *Arabidopsis thaliana*
   Tissues of 20 wild type plants of BC₂S selfing progeny derived from BC₂ plants which were generated during the work with the dwarf mutant were combined for RNA isolation. BC₂S families depicted as S1, S5, S6, S7 and S8 are related. ETID was performed with 3' end *En/Spm* primers (A) or with 5' end *En/Spm* primers (B), respectively. Products separated on 2.5% Small Low Melt agarose (BIOZYM) gels were isolated for sequencing. The prominent bands of S5, S6 and S7 correspond to the C5, C6 and C78 fragments, respectively, and are framed. In lane L, only the 100 bp and 800 bp fragments of the 100 Base-Pair Ladder of Amersham Pharmacia Biotech are depicted.
Figure 3
   Inverse PCR for the isolation of flanking sequences of the C6 insertion found by ETID
   Aliquots of genomic DNA digested with 6 enzymes which do not cut within the *En/Spm* sequence were combined and fragments longer than 8 kb were isolated from a gel and circularized. These were digested with BamHI, BglII, BsiWI or HpaI for linearization as indicated in the figure and subjected to 20 cycles of long PCR with the En205R and En8130 transposon primers. PCR was repeated with the En203R and En8136 nested transposon primers or with the En8136 and EnC6-3 allele-specific primers. This allowed to identify the 2.4 kb fragment (arrow) corresponding to the C6 insertion.

### REFERENCES

Bellen, H.J., O'Kane, C.J., Wilson, C., Grossniklaus, U., Pearson, R.K. and Gehring, W.J. (1989) P-element-mediated enhancer detection: A versatile method to study development in Drosophila. Genes & Development 3(9), 1288-1300.

Bennetzen, J.L., Swanson, J., Taylor, W.C., and Freeling, M. (1984). DNA insertion in the first intron of maize *Adhl* affects message levels: cloning of progenitor and mutant *Adhl* alleles. Proceedings of the National Academy of Sciences of the United States of America 81, 4125-4128.

Chenchik, A., Zhu, Y., Diatchenko, L., Li, R., Hill, J., and Siebert, P. (1998). Generation and use of high-quality cDNA from small amounts of total RNA by SMART PCR. In: RT-PCR Methods for Gene Cloning and Analysis; P. Siebert and J. Larrick, eds.; BioTechniques Books), pp. 305-319.

Choe, S., Dilkes, B.P., Fujioka, S., Takatsuto, S., Sakurai, A., and Feldmann, K.A. (1998). The DWF4 gene of Arabidopsis encodes a cytochrome P450 that mediates multiple 22-alpha-hydroxylation steps in brassinosteroid biosynthesis. Plant Cell *10,* 231-243.

Chomczynski, P. (1992). Solubilization in formamide protects RNA from degradation. Nucleic Acids Research 20, 3791-3792.

Earp, D.J., Lowe, B., and Baker, B. (1990). Amplification of genomic sequences flanking transposable elements in host and heterologous plants - a tool for transposon tagging and genome characterization. Nucleic Acids Research *18*, 3271-3280.

Edwards, K., Johnstone, C., and Thompson, C. (1991). A simple and rapid method for the preparation of plant genomic DNA for PCR analysis. Nucleic Acids Research 19, 1349.

Frey, M., Stettner, C., and Gierl, A. (1998). A general method for gene isolation in tagging approaches: Amplification of insertion mutagenised sites (AIMS). Plant Journal *13,* 717-721.

Frohman, M.A., Dush, M.K., and Martin, G.R. (1988). Rapid production of full-length cDNAs from rare transcripts: amplification using a single gene-specific oligonucleotide primer. Proceedings of the National Academy of Sciences of the United States of America *85,* 8998-9002.

Gaillard, C., and Strauss, F. (1990). Ethanol precipitation of DNA with linear polyacrylamide as carrier. Nucleic Acids Research 18, 378.

Hirochika, H. (1997). Retrotransposons of rice: their regulation and use for genome analysis. Plant Mol. Biol. 35, 231-240.

Liu, Y.G., Mitsukawa, N., Oosumi, T., and Whittier, R.F. (1995). Efficient isolation and mapping of Arabidopsis thaliana T-DNA insert junctions by thermal asymmetric interlaced PCR. Plant Journal 8, 457-463.

Lucas, H., Feuerbach, F., Kunert, K., Grandbastien, M.A., and Caboche, M. (1995). RNA-mediated transposition of the tobacco retrotransposon Tntl in Arabidopsis thaliana. EMBO Journal 14, 2364-73.

McClintock, B. (1954). Mutations in maize and chromosomal aberrations in Neurospora. Carnegie Inst. Washington Year Book *53,* 254-260.

Ochman, H., Ayala, F.J., and Hartl., D.L. (1993). Use of polymerase chain reaction to amplify segments outside boundaries of known sequences. In: Methods in Enzymology, R. Wu, ed. (San Diego, California, USA; London, England, UK.: Academic Press, Inc.), pp. 309-321.

Pereira, A., and Aarts, M.G.M. (1998). Transposon tagging with the En-I system. In: Arabidopsis protocols, J.M. Martinez Zapater and J. Salinas, eds. (Totowa, New Jersey, USA; Humana Press, Inc.), pp. 329-338

Pereira, A., Cuypers, H., Gierl, A., Schwarz-Sommer, Z., and Saedler, H. (1986). Molecular analysis of the *En/Spm* transposable element system of *Zea mays.* EMBO 5, 835-841.

Peterson, P. (1953). A mutable pale green locus in maize. Genetics *38,* 682-683.

Plasterk, R.H.A., Izsvák, Z., and Ivics, Z. (1999). Resident aliens: the Tc1/mariner superfamily of transposable elements. Trends in Genetics *15,* 326-332.

Sato, Y., Sentoku, N., Miura, Y., Hirochika, H., Kitano, H., and Matsuoka, M. (1999). Loss-of-function mutations in the rice homeobox gene OSH15 affect the architecture of internodes resulting in dwarf plants. EMBO Journal 18, 992-1002.

Schaefer, B.C. (1995). Revolutions in rapid amplification of cDNA ends: New strategies for polymerase chain reaction cloning of full-length cDNA ends. Analytical Biochemistry 227, 255-273.

Schwarz-Sommer, Z., Leclercq, L., Goebel, E., and Saedler, H. (1987). Cin4 an insert altering the structure of the A1 gene in zea mays exhibits properties of nonviral retrotransposons. EMBO 6, 3873-3880.

Shagin, D.A., Lukyanov, K.A., Vagner, L.L., and Matz, M.V. (1999). Regulation of average length of complex PCR product. Nucleic Acids Res. 27, e23.

Spradling, A.C., Stern, D.M., Kiss, I., Roote, J., Laverty, T. & Rubin, G.M. (1995). Gene disruptions using P transposable elements: An integral component of the Drosophila Genome Project. Proc. Natl. Acad. Sci. USA 92(24), 10824-10830.

Van, D.B.D., Maes, T., Sauer, M., Zethof, J., De, K.P., D'Hauw, M., Van, M.M., and Gerats, T. (1998). Transposon display identifies individual transposable elements in high copy number lines. Plant Journal *13,* 121-129.

van Doorn, L.J., van Belkum, A., Maertens, G., Quint, W., Kos, T., and Schellekens, H. (1992). Hepatitis C virus antibody detection by a line immunoassay and (near) full length genomic RNA detection by a new RNA-capture polymerase chain reaction. Journal of Medical Virology *38,* 298-304.

Walbot, V. (1992). Strategies for mutagenesis and gene cloning using transposon tagging and T-DNA insertional mutagenesis. In: Annual Review of Plant Physiology and Plant Molecular Biology 43, W.R. Briggs, ed. (Palo Alto, California, USA: Annual Reviews Inc.), pp. 49-82.

Wisman, E., Cardon, G.H., Fransz, P., and Saedler, H. (1998). The behaviour of the autonomous maize transposable element *En/Spm* in *Arabidopsis thaliana* allows efficient mutagenesis. Plant Molecular Biology 37, 989-999.

Yang, H., Saitou, T., Komeda, Y., Harada, H., and Kamada, H. (1997). Arabidopsis thaliana ECP63 encoding a LEA protein is located in chromosome 4. Gene *184,* 83-88.

Yephremov, A., and Saedler, H. (1995). A PCR based Transposon Insertion Display (TID) for gene tagging and evolutionary studies. In 37^{th} Annual Maize Genetics Conference, B. Sheridan, ed. (Asilomar, California, USA: Univ. North Dakota, Grand Forks), pp. 69.

## Claims

1. A method for the identification of a transposon insertion within a transcribed portion of a gene of interest in an organism or tissue of interest, said method comprising the following steps:
- isolating RNA from the organism or tissue of interest;
- hybridizing the isolated RNA to one or more polymer molecule(s) containing nucleobases capable of hybridization with at least a part of the transposon sequence in a chimeric RNA gene transcript;
- performing a reverse transcription reaction primed by said polymer molecules;
- optionally amplifying the cDNA resulting from the reverse transcription reaction;
- analysing the obtained cDNA fragments by one or more of the following methods:
a) analysing the obtained cDNA fragments for segregation of fragments;
b) using the fragments as probes or targets in nucleic acid hybridization assays;
c) sequencing of the obtained cDNA fragments.

2. The method of claim 1 wherein the organism of interest is a fungus, a plant or an animal or wherein the tissue of interest is of fungal, plant or animal origin.

3. The method of claim 1 or 2 wherein the reverse transcription reaction is carried out by a PCR cDNA synthesis technique.

4. The method of claim 3 wherein the reverse transcription reaction is carried out by the 5' RACE PCR technique.

5. The method of claim 3 or 4 wherein the cDNA resulting from the reverse transcription reaction is further amplified by PCR.

6. The method of any of claims 1 to 5 comprising an additional nucleic acid capturing step for an at least partial purification of target RNA or cDNA molecules from RNA or cDNA molecules not harbouring at least a part of the transposon insertion.

7. The method of claim 6 wherein for said additional nucleic acid capturing step one or more polymer molecules containing nucleobases hybridizing to at least a part of a RNA transcript of the transposon or a cDNA transcript of the latter is/are used wherein said polymer molecules additionally carry at least one labelling molecule which may be specifically bound by a capturing molecule immobilized on a solid carrier phase.

8. The method of claim 6 wherein for said additional nucleic acid capturing step one or more polymer molecules containing nucleobases hybridizing to at least a part of a RNA transcript of the transposon or a cDNA transcript of the latter is/are used wherein said polymer molecules are immobilized on a solid carrier phase.

9. The method of claim 7 or 8 wherein, although said polymer molecule containing nucleobases is capable of hybridization to at least a part of a RNA transcript of the transposon or a cDNA transcript of the latter, said polymer molecule is not capable of priming a reverse transcription reaction.

10. The method of any of claims 6 to 9 wherein for said additional nucleic acid capturing step one or more biotin labelled polymer molecules containing nucleobases hybridizing to at least a part of a RNA transcript of the transposon or a cDNA transcript of the latter is/are used in combination with a solid carrier phase comprising immobilized streptavidin or avidin molecules.

11. The method of any of claims 1 to 10 wherein the segregation analysis of fragments is performed by gel electrophoresis and/or by Southern blot for the detection of RFLPs.

12. The method of any of claims 1 to 10 wherein the obtained cDNA fragments are used as probes in a high-throughput hybridization screening.

13. The method of any of claims 1 to 10 and 12 wherein for the analysis of the obtained cDNA fragments, said cDNA fragments are spotted on filters or chips.

14. The method of any of claims 1 to 13 wherein the obtained cDNA fragments are ligated to other nucleic acid molecules and are subsequently subjected to an amplification *in vitro* or by propagation in host organisms.

15. A method for the identification of a gene of interest susceptible to an insertion of a particular transposon within the transcribed portion of said gene, said method comprising the steps of applying the method of any one of claims 1 to 14 using one or more polymer molecules containing nucleobases capable of hybridization to at least a part of a transcript of said particular transposon and, optionally, one or more of the following steps:
hybridizing to suitable sequence specific nucleotide probes, cloning and/or sequencing of the resulting fragments for obtaining sequence information with respect to the nucleotide sequence portions of said gene flanking the transposon insertion.

16. A method for the identification of a gene within an organism or tissue of interest the transposon insertion mutation of which being related to a particular phenotype of said organism or tissue, said method comprising the steps of applying the method of any one of claims 1 to 14 using one or more polymer molecules containing nucleobases capable of hybridization to at least a part of a transcript of said transposon and, optionally, one or more of the following steps:
hybridizing to suitable sequence specific nucleotide probes, cloning and/or sequencing of the resulting fragments for obtaining sequence information with respect to the nucleotide sequence portions of said gene flanking the transposon insertion.

17. A method for the identification of a gene within an organism or tissue of interest the mutation of which being related to a particular phenotype of said organism or tissue, said method comprising the following steps:
- performing a transposon insertion mutation of said organism by use of a particular transposon;
- selecting a particular phenotype from the resulting mutated organisms;
- applying the method of any one of claims 1 to 14 to said organism or a particular tissue thereof using one or more polymer molecules containing nucleobases capable of hybridization to at least a part of a transcript of the transposon used for the transposon insertion mutation; and,
- optionally, one or more of the following steps:
hybridizing to suitable sequence specific nucleotide probes, cloning and/or sequencing of the resulting fragments for obtaining sequence information with respect to the nucleotide sequence portions of said gene flanking the transposon insertion.

18. The method of any of claims 15, 16 or 17 wherein the obtained sequence information with respect to the nucleotide sequence portions of said gene flanking the transposon insertion is used for the construction of hybridization primer(s) for the screening of an organism or tissue of interest for the unmutated gene of interest or for related homologous genes in other organisms or tissues.

19. The method of any of claims 1 to 18 wherein the transposon is any one of the transposable elements *En/Spm*, *Mu1*, *Cin4, Ac/Ds*, *Mu2 - Mu9*, *Tag1*, *Tam1 - Tam9.*

20. A kit for performing the method of any of claims 1 to 19 said kit comprising:
- one or more polymer molecules containing nucleobases capable of specific hybridization to portions of particular types of inserted transposons and/or transcripts thereof; and/or
- one or more transposable element(s) for the mutation of a target organism to be investigated with respect to the insertion of said transposable element(s); and/or
- reagents required for performing the specific reverse transcription and, optionally, also amplification of the 5' end portions of the chimeric RNAs, such as reagents required for performing 5' RACE-PCR;
- optionally, reagents required for the analysis for segregation of fragments and/or for the hybridization, cloning and/or sequencing of the obtained fragments; and
- optionally, one or more auxiliary components, such as buffers, to be used in the ETID method.


SEQUENCE LISTING

<110> Max-Planck-Gesellschaft zur Förderung der Wissenschaften e.V.

<120> Direct Isolation of Transposon Insertions Tagging
      Transcribed Portions of Genes

<130> EP-83 193

<140>
<141>

<160> 11

<170> PatentIn Ver. 2.1

<210> 1
<211> 26
<212> DNA
<213> Artificial Sequence

<220>
<223> Description of Artificial Sequence: primer En205R;
      also used in a biotinylated form as BioEn205R

<400> 1
agaagcacga cggctgtaga atagga                                      26


<210> 2
<211> 26
<212> DNA
<213> Artificial Sequence

<220>
<223> Description of Artificial Sequence: primer En203R

<400> 2
agcacgacgg ctgtagaata ggattg                                      26


<210> 3
<211> 26
<212> DNA
<213> Artificial Sequence

<220>
<223> Description of Artificial Sequence: primer En91R

<400> 3
tgcagcaaaa cccacacttt tacttc                                      26


<210> 4
<211> 25
<212> DNA
<213> Artificial Sequence

<220>
<223> Description of Artificial Sequence: primer En48R

<400> 4
tttacttcga ttaagagtgt cggcc                                       25

<210> 5
<211> 26
<212> DNA
<213> Artificial Sequence

<220>
<223> Description of Artificial Sequence: primer En8130;
      also used in a biotinylated form as BioEn8130

<400> 5
gagcgtcggt ccccacactt ctatac                                          26


<210> 6
<211> 27
<212> DNA
<213> Artificial Sequence

<220>
<223> Description of Artificial Sequence: primer En8133

<400> 6
cgtcggtccc cacacttcta tacgaat                                         27


<210> 7
<211> 28
<212> DNA
<213> Artificial Sequence

<220>
<223> Description of Artificial Sequence: primer En8136

<400> 7
cggtccccac acttctatac gaataaga                                        28


<210> 8
<211> 24
<212> DNA
<213> Artificial Sequence

<220>
<223> Description of Artificial Sequence: primer En8179

<400> 8
tgacggctaa gagtgtcggt caac                                            24


<210> 9
<211> 30
<212> DNA
<213> Artificial Sequence

<220>
<223> Description of Artificial Sequence: SMART II
      Oligonucleotide (component of the SMART PCR cDNA
      Synthesis Kit of Clontech)

<400> 9
aagcagtggt aacaacgcag agtacgcggg                                      30

<210> 10
<211> 23
<212> DNA
<213> Artificial Sequence

<220>
<223> Description of Artificial Sequence: PCR primer
      (component of the SMART PCR cDNA Synthesis Kit of
      Clontech)

<400> 10
aagcagtggt aacaacgcag agt                                          23


<210> 11
<211> 27
<212> DNA
<213> Artificial Sequence

<220>
<223> Description of Artificial Sequence: primer EnC6-3

<400> 11
ctccttacct tttttcttgt agtggct                                      27